# EUROPEAN PATENT SPECIFICATION

(11) **EP 3 074 028 B1**
(45) Date of publication and mention of the grant of the patent: **06.04.2022**
(21) Application number: 14866584.7
(22) Date of filing: 28.11.2014
(51) Int. Cl.: A61K 31/12, A61K 31/05, A61K 31/353, A61K 9/00, A61K 9/06, A61K 9/127, A61P 35/00, A61P 31/12

(54) **ACTIVITY ENHANCING CURCUMIN COMPOSITIONS AND METHODS OF USE**
AKTIVITÄTSVERSTÄRKENDE CURCUMIN-ZUSAMMENSETZUNGEN UND VERFAHREN ZUR VERWENDUNG
COMPOSITIONS DE CURCUMINE À ACTIVITÉ AMÉLIORÉE ET MÉTHODES D'UTILISATION

(30) Priority: 27.11.2013 US 201361909579 P
(43) Date of publication of application: 05.10.2016
(73) Proprietor: Research Foundation Of The City University Of New York, New York, NY 10019 (US); The Feinstein Institute for Medical Research, Manhasset, NY 11030 (US)
(72) Inventor: CASTELLANOS, Mario, R., Brooklyn, NY 11230 (US); BANERJEE, Probal, Staten Island, NY 10310 (US); DEBATA, Priya, Ranjan, New York, NY 10019 (US); SZERSZEN, Anita, Staten Island, NY 10309 (US); FATA, Jimmie, E., Staten Island, NY 10314 (US)
(74) Representative: V.O.
(86) International application number: PCT/US2014/067819
(87) International publication number: WO 2015/081319

(56) References cited:
- WO-A1-2012/068179
- WO-A1-2012/150370
- WO-A1-2013/050962
- WO-A2-2006/087759
- WO-A2-2007/103435
- WO-A2-2008/122967
- WO-A2-2008/131059
- WO-A2-2011/011721
- WO-A2-2012/142511
- JP-A- H04 264 027
- US-A1- 2002 164 385
- US-B2- 7 351 739
- US-B2- 8 383 865
- ADHIP P. N. MAJUMDAR ET AL: "Curcumin Synergizes With Resveratrol to Inhibit Colon Cancer", NUTRITION AND CANCER, vol. 61, no. 4, 17 July 2009 (2009-07-17), pages 544-553, XP055390069, US ISSN: 0163-5581, DOI: 10.1080/01635580902752262
- SOVAK M ET AL: "PSA rising syndrome: Resorption, synergism and potential utility of t-Resveratrol, its glycon piceid and Curcumin", AMERICAN ASSOCIATION FOR CANCER RESEARCH. PROCEEDINGS OF THE ANNUAL MEETING; [CANCER RESEARCH; APRIL 2004, VOLUME 64, ISSUE 7, SUPPLEMENT], AMERICAN ASSOCIATION FOR CANCER RESEARCH, US, vol. 44, no. 2ND ED, 1 July 2003 (2003-07-01), page 942, XP001246852, ISSN: 0197-016X
- AFTAB N ET AL: "Antioxidant activities of curcumin and combinations of this curcuminoid with other phytochemicals", PHYTOTHERAPY RESEARCH, WILEY, UK, 1 January 2012 (2012-01-01), pages 500-502, XP018502196, ISSN: 0951-418X, DOI: 10.1002/PTR.2960
- CSAKI CONSTANZE ET AL: "Synergistic chondroprotective effects of curcumin and resveratrol in human articular chondrocytes: inhibition of IL-1Î-induced NF-Î B-mediated inflammation and apoptosis", ARTHRITIS RESEARCH AND THERAPY, BIOMED CENTRAL, LONDON, GB, vol. 11, no. 6, 4 November 2009 (2009-11-04), page R165, XP021065266, ISSN: 1478-6354, DOI: 10.1186/AR2850
- DEBATA PRIYA RANJAN ET AL: "A novel curcumin-based vaginal cream Vacurin selectively eliminates apposed human cervical cancer cells", GYNECOLOGIC ONCOLOGY, ACADEMIC PRESS, LONDON, GB, vol. 129, no. 1, 9 December 2012 (2012-12-09), pages 145-153, XP028524503, ISSN: 0090-8258, DOI: 10.1016/J.YGYNO.2012.12.005
- "Composition for treatment of papilloma virus-infected tissue - comprises tea extract containing catechin or its derivatives, as active ingredient", DERWENT, 1996, XP002247648,

## Description

### BACKGROUND

Cervical cancer is a leading cause of cancer death amongst females worldwide, of which the human papillomavirus (HPV) is the main etiologic risk factor. In developed countries, like the U.S., cervical cancer screening programs are effective in reducing mortality. However, the annual cost to treat HPV-associated pre-cancers is in the order of billions of dollars, and therapy is predominately surgical. Therefore, despite advancements, cervical cancer and precancerous lesions of the cervix continue to be a global health issue, while an effective treatment for chronic HPV infection has remained elusive.

There is no current treatment for chronic cervical HPV infection, yet this oncogenic virus is a major cause of morbidity and mortality worldwide. HPV (+) patients cannot be offered any therapy; instead these women are enrolled in yearly surveillance programs and monitored for the development of pre-cancers or cancerous lesions.

Chronically HPV infected patients can develop high grade cervical dysplasia or cancer. Treatment relies on surgical or ablative therapies removing abnormal cells. These invasive procedures have been linked to future adverse pregnancy complications because of their effects on the cervix, leading to premature births or miscarriages.

Antitumor and antiviral agents have been investigated for treatment of HPV, including 5-flurouracil or imiquimod. However, these agents produce toxic effects on non-diseased cells in the vaginal and cervical epithelium and are not consistently effective.

The global disease burden of HPV infection and associated cancers is enormous. Many women are affected in both developing and developed nations. Sophisticated technologies like gene therapy, therapeutic cancer vaccines, and synthetic antivirals are some of the approaches being examined as possible new therapies. However, none of these have been consistently effective in clinical trials. Even if some of these strategies can be developed to show some promise, they are not able to be used in all regions of the world because the high cost associated with manufacturing these more complex technologies.

Hence there exists a long standing need to provide a therapeutic agent for the treatment of oncogenic viruses and oncogenic conditions that do not harm healthy cells, is easily manufactured or produced, and less costly.

### SUMMARY OF THE INVENTION

Over millennia plants have evolved strong defenses against pathogenic microbes, including viruses. A plant's immune system is mediated by chemical messengers. Polyphenols are an important class of compounds that have allowed some plant species to thrive. Some important polyphenols include catechins from green tea, and resveratrol from grapes. These small molecules activate potent mechanisms that fend off disease. Plant polyphenols can affect cellular processes in humans as there is shared homology in biochemical pathways in among species. Curcumin, a yellow pigment of the food spices turmeric, is a phenolic compound known for its anti-inflammatory and anti-tumor effects. Though extremely promising in laboratory studies, many plant polyphenols have not shown major therapeutic efficacy in human clinical trials. Curcumin is a prime example of a plant polyphenol with disappointing clinical trial results. One reason for the disappointing clinical trial results is due to low systemic bioavailability because curcumin is rapidly cleared from the blood; as a result it does not reach the target tissue in high enough concentrations.

, Herein disclosed are two polyphenols, when present in distinct amounts that interact synergistically with curcumin to lower its IC50. This synergy surprisingly increases the target tissue effect of curcumin. More in particular, the invention provides a pharmaceutical composition comprising curcumin, catechin and resveratrol, wherein said curcumin, catechin and resveratrol are present in a ratio of 32:8:100, and wherein said catechin is (-)-epicatechin or a gallic acid conjugate thereof. Furthermore, it has been unexpectedly discovered that this triple combination has dramatic effects in an *in vivo* mice model. It has been unexpectedly discovered to activate new genes in microarray analysis, which are not activated when each compound is used alone. Finally, it has been unexpectedly discovered that the two polyphenols provide enhanced cellular absorption of curcumin and can stabilize curcumin in an aqueous environment.

Any reference in the description to methods of treatment or use in a method of treatment refer to compounds, pharmaceutical compositions and medicaments of the present invention for use in a method for treatment of the human or animal body by therapy.

In one embodiment, the present invention provides for compositions and formulations with functions not previously known including a curcumin, and at least one of resveratrol and catechin. The present invention also provides for the use of compositions in a method of treating any disease in which uncontrolled cellular proliferation occurs, such as cancers. In particular, the present invention provides for the use of compositions in a method for treating cervical cancer and precancerous cervical lesions. The present invention also provides for the use of compositions in a method for treating viral infections.

### BRIEF DESCRIPTION OF THE DRAWINGS

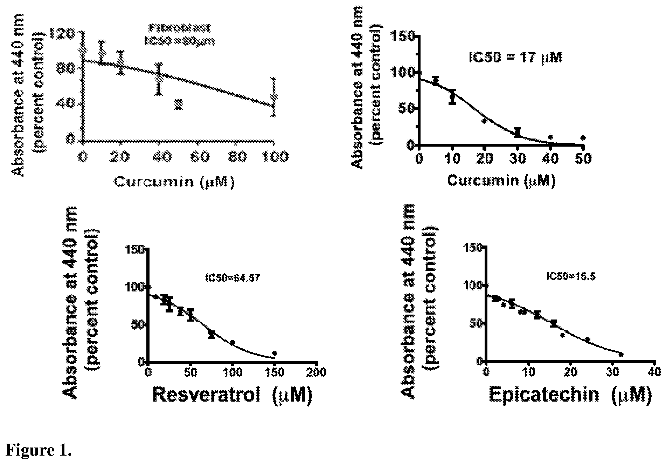

Figure 1 depicts the independent effects of Curcumin, Epicatechin, and Resveratrol on HeLa cells in a dose dependent manner. The effects of Curcumin on primary human fibroblasts are also shown. Cells were treated with indicated concentrations of each compound for 96 hours and then subjected to WST-1 assays. Carrier-treated HeLa cells were used as control. The data were analyzed using the Graphpad Prism software. The IC50 value for each drug is indicated in the graph.

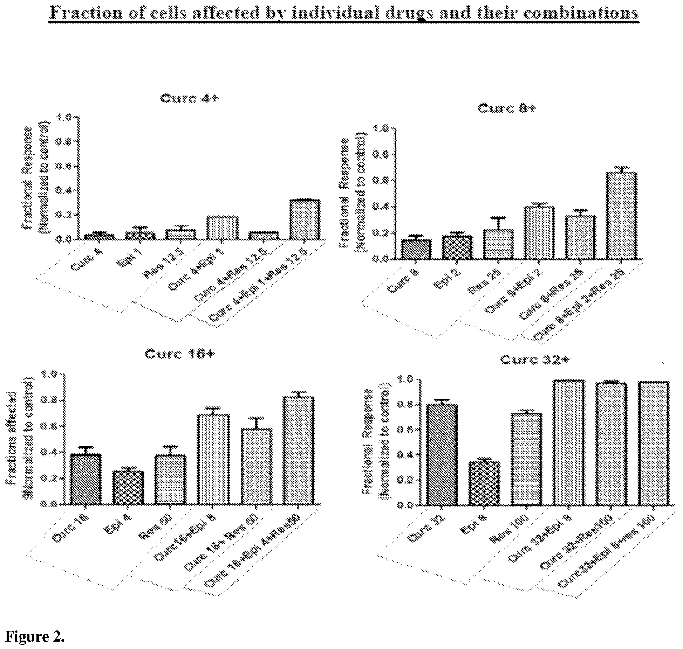

Figure 2 depicts synergistic anti-cancer activities of Curcumin, Epicatechin, and Resveratrol combinations on HeLa cells. Curcumin, Epicatechin, and Resveratrol alone or in combinations were used to treat HeLa cells at indicated doses. After 96 hours of treatment, WST-1 assay was carried out to estimate cell viability. Carrier-treated cells were used as control. The fractions of cells affected by these treatments were plotted using Graphpad Prism. Results demonstrate that the combinations of the three anti-cancer agents, at specified ratios, have better killing activities than the individual drugs or the combinations of two drugs. The graphs represent fraction affected (FA) and the Combination Index (CI) using HeLa cells.

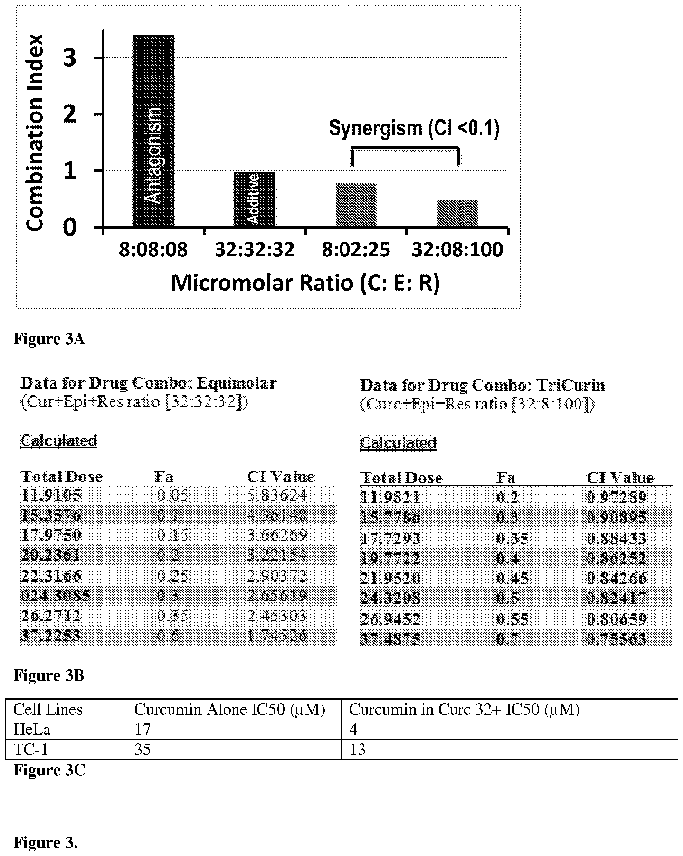

Figure 3A depicts Combination Index values for varying ratio/concentrations of Curcumin (C), Epicatechin (E), and Resveratrol (R). The data show that Curcumin (C), Epicatechin (E), and Resveratrol (R) in Curc 32+ show synergism, which is not displayed by the same components in an equi-molar combination. Combination index (CI) values above 1 indicate antagonism and CI = 1 indicates additive effect in the equimolar ratios of CER. In contrast, the composition of the present invention wherein curcumin is present at both 8 µM+(8:2:25) and 32 µM+(32:8:100) show synergism (CI < 1).
Figure 3B depicts percentage of HeLa cells killed (Fraction Affected=FA) by similar doses of equimolar ratio and Curc 32+and combination indices showing synergism only for Curc 32+. At equal doses, the combination of equimolar C+E+R is always antagonistic and kills much lower number of HeLa cells. In contrast, when equal doses of C+E+R are used in the derived ratio of 32:8:100, the Combination Index shows synergy and the Fa is much higher for Curc 32+.
Figure 3C depicts a table showing the IC50 values for curcumin alone or in the Curc32+ form. Thus, while the IC50 values for curcumin alone is 17 µM and 35 µM for HeLa and TC-1 cells, respectively, the IC50 values for curcumin in Curc 32+ are 4 µM and 13 µM for HeLa and TC-1 cells respectively.

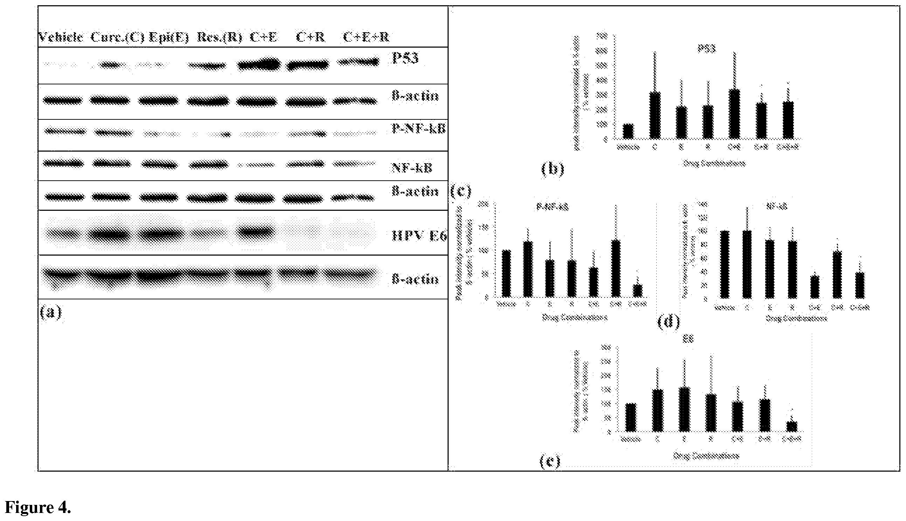

Figure 4 depicts Curcumin, Epicatechin, and Resveratrol combinations on cellular various protein levels. A combination of Curcumin, Epicatechin, and Resveratrol causes rapid suppression of Phospho-NFkB (P-NFkB), NFkB, P-Rb, HPV-E6 and restoration of p53 in HeLa cells in 8 hours. (a) Post-treatment, whole cell lysates were analyzed by Western blot analysis (30 µg/lane). (b-e) The quantification of bands from three independent experiments was performed using ImageJ program and normalized against beta-actin intensity. Results obtained from each treatment group were converted to percent vehicle-treated and then compared with the vehicle treated (using student's t-test). In each case, C+E+R (Curc 32+) produced a significant change in band intensity with respect to the vehicle-treated samples (^{∗}p < 0.05).

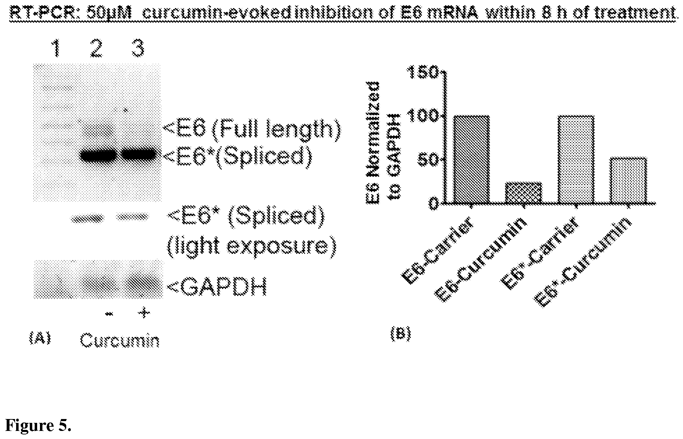

Figure 5 depicts E6 mRNA levels in HeLa cells treated with curcumin. Curcumin treatment for 8 hours dramatically inhibits E6 transcription. HeLa cells were cultured in 6 well plates and treated with either carrier or curcumin (50 µM) for 8 h. RNA was isolated using the Trizol reagent and treated with DNase I to remove contaminating DNA. Using primers specific for HPV18 E6 or GAPDH, semi-quantitative reverse transcriptase PCR (RT-PCR) was performed on cDNA samples obtained from both treatments. Equal volumes of the PCR reactions were separated on a 1.5 % agarose-ethidium bromide gel. (A) Two amplified products were obtained- one of ~450 bps corresponding to full-length E6 and a second of ~ 280 bps representing the spliced E6^{∗} product. A low exposure image of the spliced PCR products is also shown below. (B) The intensity if each band was quantified, normalized to GAPDH, and plotted in graphs.

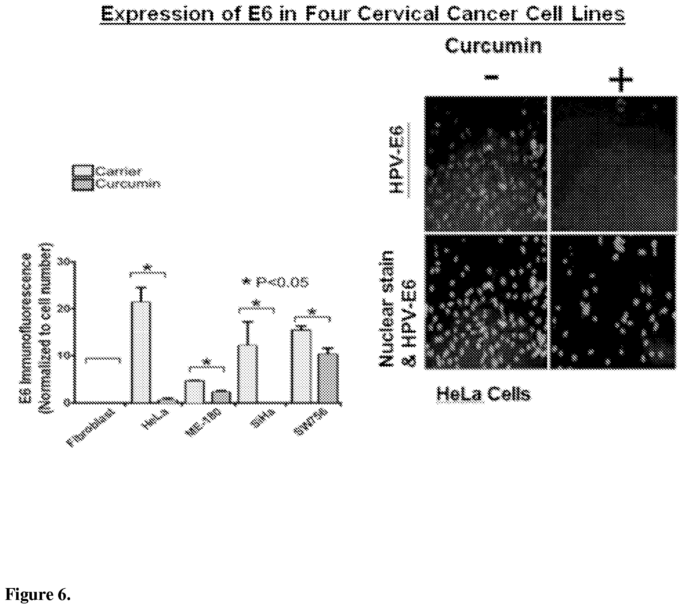

Figure 6 depicts expression of E6 in four cervical cancer cell lines. Immunocytochemical analysis reveals detectable expression of the HPV oncogenic protein E6 in all four cervical cancer cell lines, HeLa, ME-180, SiHa, and SW756, but not in the human fibroblast cells (only graph is shown). Curcumin (50 µM) treatment caused a significant decrease in the expression of HPV-E6 in all the four cell lines (only HeLa cells images are shown); demonstrating curcumin has antiviral properties reversing the overexpression of the oncogenic protein E6.

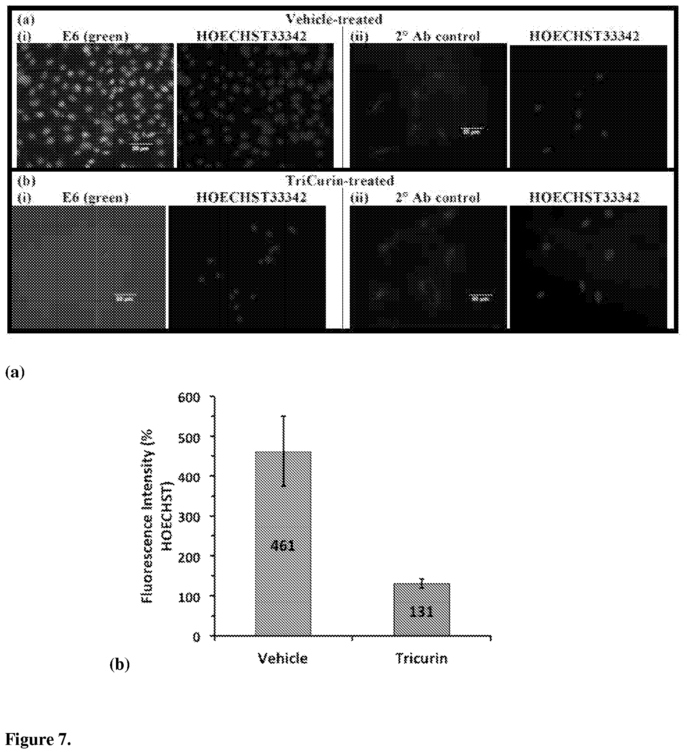

Figure 7 depicts E6 expression in HPV(+)tumor cells treated with Curc 32+. (a) Vehicle-treated TC-1 cells show high level of E6 expression (i). Lack of staining with the 2° antibody alone confirms the specificity of the E6 primary antibody (a ii). **(b)** Upon 24 hours of Curc 32+ treatment, the E6 expression is completely suppressed in these cells (i). No staining is again observed with the 2° antibody alone (ii). **(c)** Quantification of E6 fluorescence intensity from triplicate samples after normalizing to the intensity of HOECHST confirmed a dramatic inhibition of E6 expression upon Curc 32+ treatment.

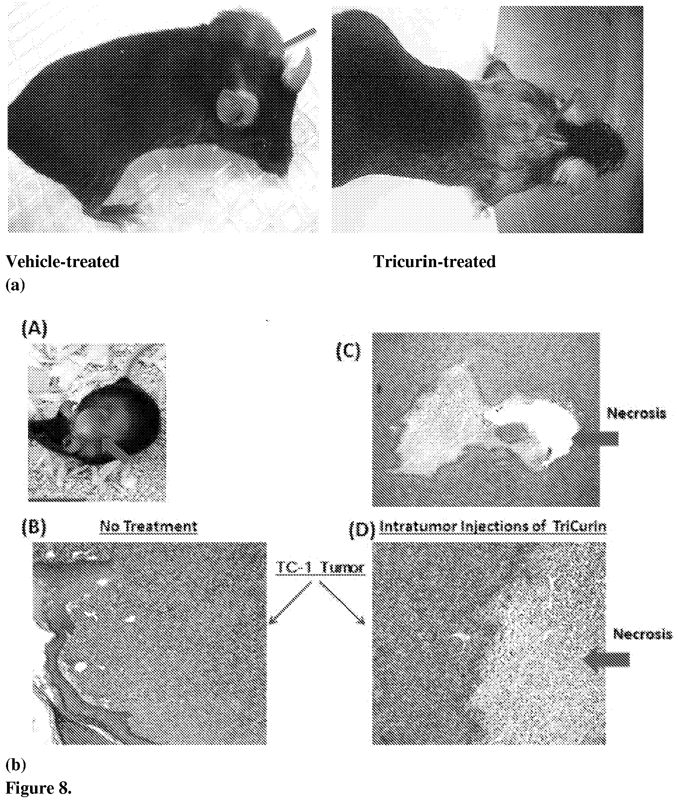

Figure 8 depicts intra-tumor administration of Curc 64+ in mice. **(a)** C57BL/6 mice had 50,000 TC-1 cancer cells implanted in the neck of each mouse. Neck tumors cause death at 4-5 weeks. For the intralesional experiments, TC-1 tumors were allowed to grow for about 2 weeks then mice received two doses of vehicle (0.1 DMSO in PBS) or Curc 64+ 72 hours apart. The treated mouse shows dramatic regression of tumor 48 hours after the second treatment. **(b) (A)** C57BL/6 mice had 50,000 TC-1 cancer cells implanted in the neck of each mouse. Neck tumors cause death at 4-5 weeks. For the intralesional experiments, TC-1 tumors were allowed to grow for about 2 weeks then mice received two doses of Curc 32+ 72 hours apart. Mice were sacrificed 5 days after last dose. **(B)** H&E stained section of TC-1 tumor in a mice with no treatment, HPV(+) carcinoma cells form a solid mass with no areas of necrosis. **(C)** Mice treated with two doses of intralesional Curc 32+ shows extensive area of tumor necrosis near the injection track, 5 days post treatment (tissue loss and extended necrosis). **(D)** High magnification image of (C) confirms the presence of tumor cell necrosis. Longitudinal experiments are ongoing to evaluate optimal dose to induce tumor regression in this aggressive cancer. Studies will include evaluation of inflammatory infiltrate to evaluate how Curc 32+ modulates pro-cancer immune response, as indicated in the microarray data.

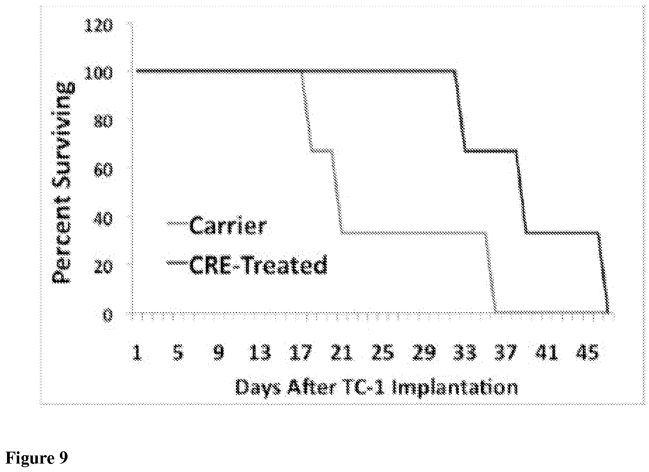

Figure 9 depicts the effect of systemic administration of a Curc 32+ on survival of mice implanted with TC-1 tumors in the neck. CRE is Curc 32+.

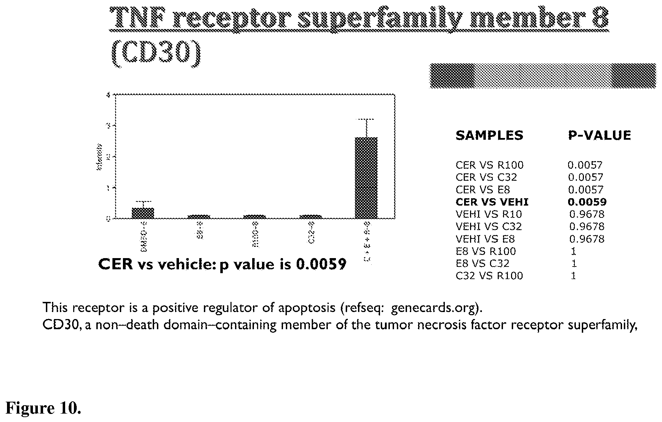

Figure 10 depicts TNF receptor superfamily member 8 (CD30) gene expression of HeLa cells treated with vehicle (0.1% DMSO in serum-free medium, depicted as DMSO), epicatechin (E), resveratrol (R), curcumin (C), or Curc 32+ (C-E-R). This receptor is a positive regulator of apoptosis. CD30 is a non-death domain-containing member of the tumor necrosis factor receptor superfamily.

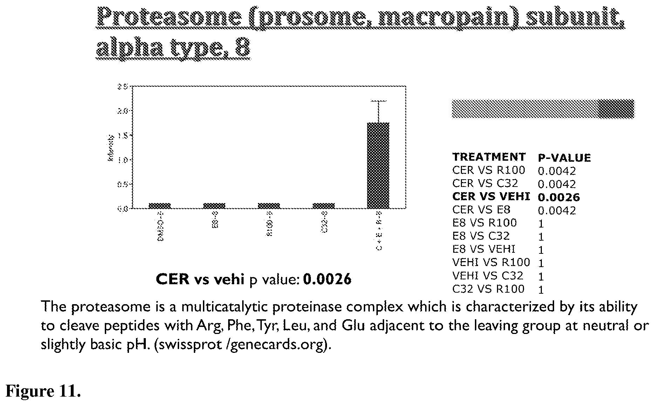

Figure 11 depicts proteasome (prosome, macropain) subunit alpha type 8 gene expression of HeLa cells treated with vehicle (0.1% DMSO in serum-free medium, depicted as DMSO), epicatechin (E), resveratrol (R), curcumin (C), or Curc 32+ (C-E-R). The proteasome is a multicatalytic proteinase complex which is characterized by its ability to cleave peptides with Arg, Phe, Tyr, Leu, and Glu adjacent to the leaving group at neutral or slightly basic pH.

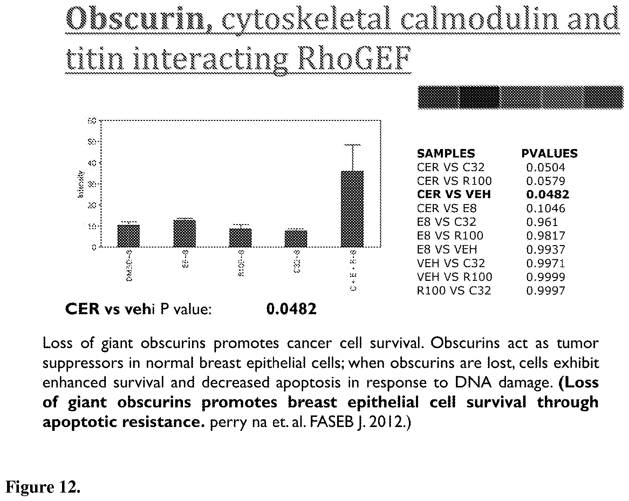

Figure 12 depicts obscurin, cytoskeletal calmodulin and titin interacting RhoGEF gene expression of HeLa cells treated with vehicle (0.1% DMSO in serum-free medium, depicted as DMSO), epicatechin (E), resveratrol (R), curcumin (C), or Curc 32+ (C-E-R). Loss of giant obsucins promotes cancer survival. Obscurins act as tumor suppressors in normal breast epithelial cells; when obscurins are lost; cells exhibit enhanced survival and decreased apoptosis in response to DNA damage.

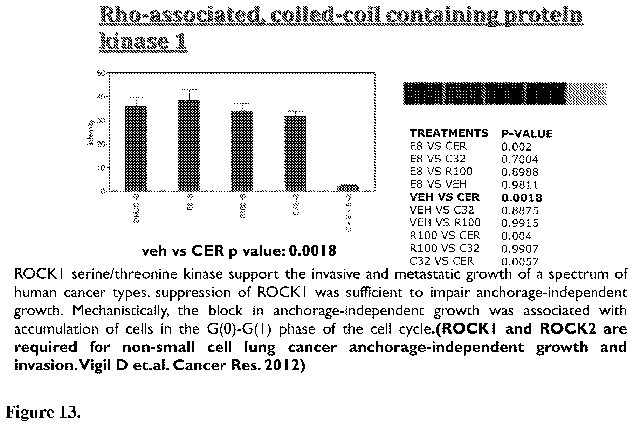

Figure 13 depicts Rho-associated, coiled-coil containing protein kinase 1(ROCK 1) gene expression of HeLa cells treated with vehicle (0.1% DMSO in serum-free medium, depicted as DMSO), epicatechin (E), resveratrol (R), curcumin (C), or Curc 32+ (C-E-R). ROCK1 serine/threonine kinase supports the invasive and metastatic growth of a spectrum of human cancer types. Suppression of ROCK 1 was sufficient to impair anchorage-independent growth. Mechanistically, the block in anchorage-independent growth was associated with accumulation of cells in the G(0)-G(1) phase of the cell cycle.

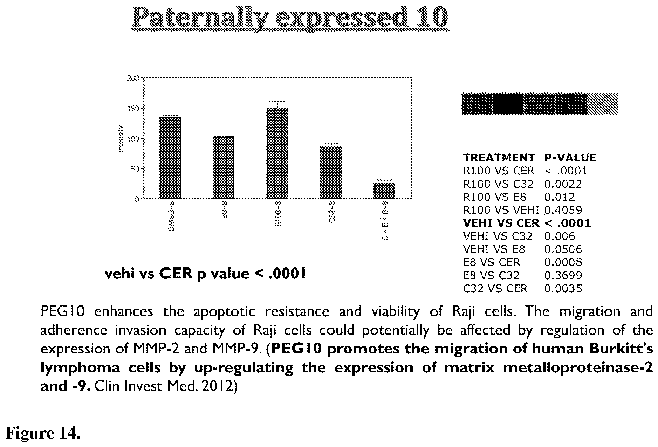

Figure 14 depicts paternally expressed 10 (PEG10) gene expression of HeLa cells treated with vehicle (DMSO), epicatechin (E), resveratrol (R), curcumin (C), or Curc 32+ (C-E-R). PEG 10 enhances the apoptotic resistance and viability of Raji cells. The migration and adherence invasion capacity of Raji cells could potentially be affected by regulation of the expression of MMP-2 and MMP-9.

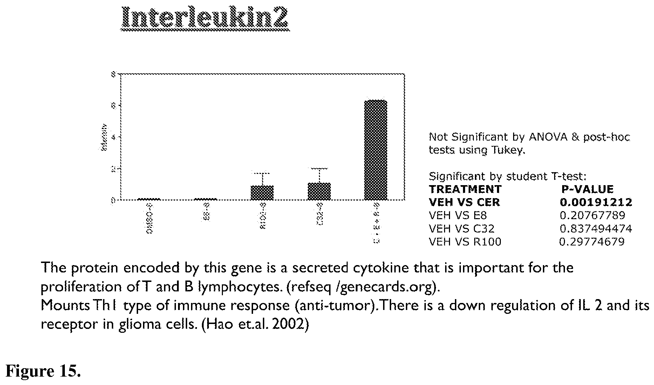

Figure 15 depicts interleukin 2 gene expression of HeLa cells treated with vehicle (0.1% DMSO in serum-free medium, depicted as DMSO), epicatechin (E), resveratrol (R), curcumin (C), or Curc 32+ (C-E-R). The protein encoded by this gene is a secreted cytokine that is important for the proliferation of T and B lymphocytes.

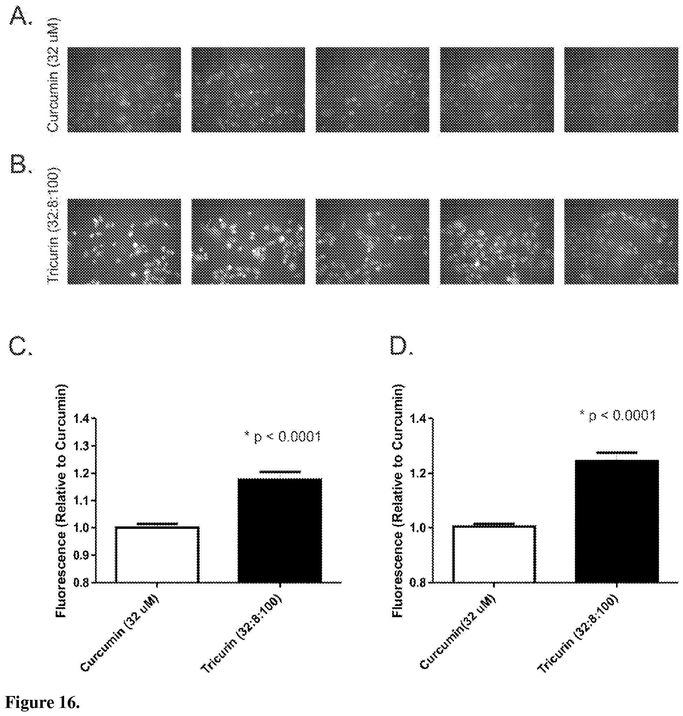

Figure 16 depicts the result of an experiment wherein cells were incubated for four hours with either curcumin (32 µM) or Curc 32+ (32:8:100). Following incubation, cellular uptake of curcumin was captured using fluorescent imaging (A and B). Cells incubated with Curc 32+ displayed significantly higher fluorescence than cells incubated with curcumin alone (p < 0.0001; t-test) (C). The net increase in curcumin uptake from Curc 32+ was was 17.8% in 4 hours. No fluorescence was noted with epicatechin or resveratrol. The fluorescence of curcumin was measured using excitation at 450 nm. When curcumin and Curc 32+were left in solution overnight at 37 °C and then added to cells at similar conditions as the 4 hour experiment, the cellular uptake of curcumin was noted to be approximately 25% higher from Curc 32+ compared with curcumin alone (p < 0.0001; D). In summary these results indicate that Curc 32+ enhances cellular uptake of curcumin and, without wishing to be bound by theory, may protect curcumin from oxidation while in solution overnight, as equal amounts of curcumin were used in the 4 and 24 hour experiments.

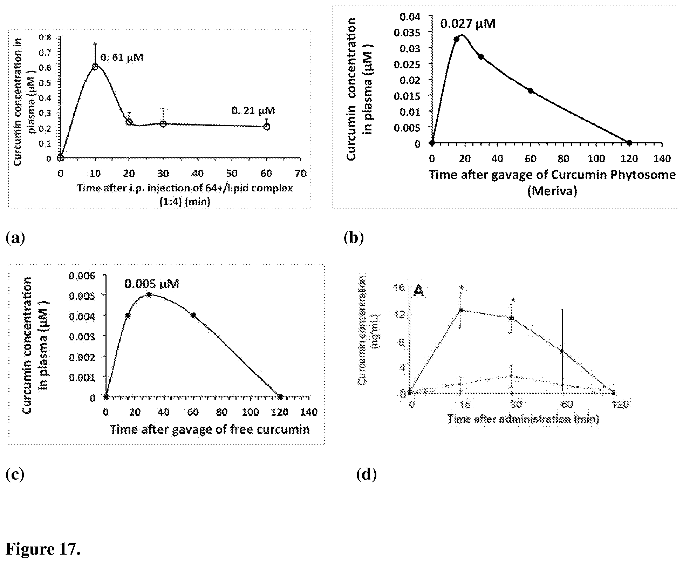

Figure 17 depicts curcumin concentration in plasma produced after I.P. injection of lipid encapsulated Curc 64+ ("64+"). This preparation and type of delivery yields ten times higher curcumin concentration in the plasma (a) than that produced by gavage of Curcumin Phytosome (Meriva) (Doctor's Best)- a lipid-encapsulated version of curcumin alone (b) and 100 times higher than that produced by gavage of free curcumin (c). Data in (b) and (c) were derived from reported data shown in (d) (Marczylo TH, Verschoyle, R.D., Cooke, D.N., Morazzoni, P., Steward, W.P., and Gescher, A.J. (2007) Comparison of systemic availability of curcumin with that of curcumin formulated with phosphatidylcholine. Cancer Chemother Pharmacol 60:171-177). Since Figures 17 (a) and (b) both show lipid-encapsulated preparations, this piece of data strongly indicates that Curc 64+ serves to stabilize curcumin in the plasma by a factor of 10 over free curcumin.

### DETAILED DESCRIPTION

Human papillomavirus (HPV) causes cervical, oropharyngeal, anal, penile, and vaginal cancer. In the US alone, HPV infects over 20 million people. In women, cancer screening identifies 300,000 cases of cervical precancers each year, higher than lung or breast cancer rates. Meanwhile, oral HPV infection is on the rise affecting millions. Men are three times more likely to get infected than women. The major risk factor for head and neck cancer is now HPV. Despite advancements, a cure to eradicate HPV infection and specifically treat these associated cancers has not been found.

Like many human viruses, HPV have existed and evolved over millions of years. They are able to grow and replicate while efficiently evading intracellular mechanism the host immune system. HPV is particularly difficult to treat as it can become latent and exist in the basal epithelial cells for years or decades. In this state HPV is episomal with low viral copies, sometimes with 1-2 copies per cell. In cancer HPV often integrates its DNA into the host cell. Classic antiviral and chemotherapy drugs works by targeting one cellular pathway. Even when multiple drugs are used, resistance can develop. HPV can exist in several states and affect a wide range of pathways. These are some of the reasons why it's been hard to eradicate HPV and HPV (+) cancers. Unlike synthetic drugs, plant polyphenols are extremely potent compounds and diverse in function.

Curcumin eliminates HPV (+) cancer cells by inducing apoptosis and by inhibiting the HPV oncoproteins E6. Furthermore, curcumin's effects were not type specific as it eliminated HPV types 16, 18, 67 cancer cell lines. Though very promising, the use of curcumin in human trials has had very limited success. This in part has been due to curcumin's low solubility in water, causing poor systemic bioavailability, rapid clearance from the blood, all together leading to low target tissue effects. The prior art has not been able to successfully teach how to use curcumin.

The present disclosure provides a novel method to enhance the effects of curcumin by identifying other plant compounds that chemically and biologically interact with each other such that are the compositions of the present disclosure, at an optimized ratio provided in the present disclosure, have potent and synergistic antiviral and anticancer properties. In addition, the disclosure provides that addition of the other compounds enhance curcumin's cellular absorption and stabilize curcumin in aqueous environment. Furthermore, the present disclosure provides microarray analysis of over 30,000 genes reveal that the triple combination interferes with known cancer cell pathways utilized to survive and hide from the immune system, which are not affected when each compound is used alone. The compositions of the present invention targets the wide range of pathways that HPV infection and associated cancers uses to evade, replicate and induce disease. The present disclosure provides that both cell culture and animal data showing the combinations of the disclosure are potent, non-toxic and highly effective.

The present disclosure relates to compositions and formulations that include a curcumin, resveratrol and a catechin. Provided is a pharmaceutical composition comprising curcumin, catechin and resveratrol, wherein said curcumin, catechin and resveratrol are present in a ratio of 32:8:100, and wherein said catechin is (-)-epicatechin or a gallic acid conjugate thereof. The present invention also provides the use of such composition in a method to reduce, prevent, suppress, or inhibit the growth or proliferation of cancer cells by administering the compositions as described herein. The present disclosure also provides the use of such composition in a method to treat, prevent, or suppress viral diseases and similar disease states by administering the compositions or formulations according to the disclosure.

A triple component composition according to claim 1 is not disclosed or suggested in the art.

Majumbar et al. (Nutrition and Cancer, 2009, Vol. 61, no. 4, pp. 544-553) teaches that curcumin synergizes with resveratrol to inhibit colon cancer. WO2006/087759A2 and Sovak et al. (Am. Assoc. Cancer Res. 2003, vol. 44, pg. 942) disclose pharmaceutical compositions comprising curcumin and resveratrol for use in treating prostate cancer. Aftab et al. (Phytotherapy Res. 2012, pp. 500-502) teaches that curcumin and resveratrol together result in a synergistic antioxidant effect. Constanze et al. (Arthritis Res. and Ther. 2009. Vol. 11, no. 6, pg. R165) relates to synergistic chondroprotective effects of curcumin and resveratrol. WO2007/103435A2 discloses compositions comprising curcuminoid, antioxidant, water-soluble pharmaceutically acceptable carrier and optionally glucuronidation inhibitor.

Whereas WO2012/142511A2 discloses an orthomolecular composition comprising, among others, the three active ingredients of the present invention, it only generally teaches that each of them can be present in an amount of "about 0.025% to about 2.5%‴. It fails to teach or suggest a pharmaceutical composition comprising the triple combination of curcumin, catechin and resveratrol in the relative ratio of the present claims.

US2002/164385A1 discloses several classes of HPV protein expression inhibitors for the treatment of HPV mediated diseases. Inhibitors comprise naturally occurring polyphenolic compounds, including curcumin, resveratrol and epigallocatechin. Notably however, it does not teach or suggest to use these compounds in combination with each other, let alone in the ratio of present claims.

It has been unexpectedly and surprisingly discovered that (-)-epicatechin gallate, derived from green tea extract and resveratrol, derived from grapes, are very effective in killing HPV (+) HeLa cells. Furthermore, the present disclosure provides that the combination of curcumin, (-)-epicatechin gallate and resveratrol, in a specific ratio are synergistic and provide potent antitumor and antiviral activity. For example, the instant disclosure shows that the IC50 of curcumin can be reduced by 3-fold when adding epicatechin gallate and resveratrol. The IC50 of epicatechin gallate can be reduced by 9-fold by adding curcumin and resveratrol. The IC50 of resveratrol can be decreased by 6-fold by adding curcumin and epicatechin. These observations are supported by cytotoxicity experiments and by intracellular signaling data presented herein. See Figures 1, 2, and 3.

The disclosure provides a novel pharmaceutical composition or formulation including curcumin-epicatechin-resveratrol, having potent antiviral and antitumor effects.

Curcumin is commonly known in the art.

As defined herein, catechin includes (-)-epicatechin as well as its gallic acid conjugates are suitable for use in the present invention.

In a preferred embodiment, the minimum average concentration of curcumin is 4 µM. In a more preferred embodiment, the minimum average concentration of curcumin is 8 µM; even more preferably, the minimum concentration of curcumin is 16 µM.

In a preferred embodiment, the maximum average concentration of curcumin is 16 µM. In a more preferred embodiment, the maximum average concentration of curcumin is 32 µM; even more preferably, the maximum concentration of curcumin is 64 µM. In another preferred embodiment, the maximum average concentration of curcumin is 100 µM.

In a preferred embodiment, the minimum average concentration of catechin is 1 µM. In a more preferred embodiment, the minimum average concentration of catechin is 2 µM; even more preferably, the minimum concentration of the catechin 4 µM.

In preferred embodiment, the maximum average concentration of catechin is 30 µM. In another preferred embodiment, the maximum average concentration of catechin is 16 µM. In a more preferred embodiment, the maximum average concentration of catechin is 8 µM; even more preferably, the maximum concentration of catechin is 4 µM.

Resveratrol is commonly known in the art. Resveratrol (3,4',5 trihydroxystilbene; CAS: 501-36-0) is a stilbenoid, a derivate of stilbene, and has a structural skeleton comprised of two aromatic rings linked by an ethylene bridge.

The compositions or formulations of the present invention comprise curcumin, catechin, and resveratrol, wherein said catechin is (-)-epicatechin or a gallic acid conjugate thereof.

In a preferred embodiment, the minimum average concentration of curcumin is 4 µM. In a more preferred embodiment, the minimum average concentration of curcumin is 8 µM; even more preferably, the minimum concentration of curcumin is 16 µM.

In a preferred embodiment, the maximum average concentration of curcumin is 16 µM. In a more preferred embodiment, the maximum average concentration of curcumin is 32 µM; even more preferably, the maximum concentration of curcumin is 64 µM. In another preferred embodiment, the maximum average concentration of curcumin is 100 µM.

In a preferred embodiment, the minimum average concentration of the catechin is 1 µM. In a more preferred embodiment, the minimum average concentration of catechin 2 µM; even more preferably, the minimum concentration of catechin is 4 µM.

In preferred embodiment, the maximum average concentration of catechin is 30 µM. In another preferred embodiment, the maximum average concentration of catechin is 16 µM. In a more preferred embodiment, the maximum average concentration of catechin is 8 µM; even more preferably, the maximum concentration of catechin is 4 µM.

In a preferred embodiment, the minimum average concentration of resveratrol is 12.5 µM. In a more preferred embodiment, the minimum average concentration of resveratrol is 25 µM; even more preferably, the minimum concentration of the resveratrol is 50 µM.

In a preferred embodiment, the maximum average concentration of resveratrol is 500 µM. In a preferred embodiment, the maximum average concentration of resveratrol is 200 µM. In a more preferred embodiment, the maximum average concentration of resveratrol 100 µM; even more preferably, the maximum concentration of resveratrol is 50 µM.

The curcumin, catechin, and resveratrol are present in the ratio 32:8:100 (as used herein "Curc 32+" or "Tricurin").

In addition, it was also unexpectedly discovered that the combination of catechin, and resveratrol with curcumin increase the cellular uptake of curcumin (Figure 16). Neither catechin nor resveratrol has been previously shown to have these effects on curcumin. Disclosed herein is that the compositions of the present invention unexpectedly function biologically to produce synergy with curcumin potentiating its effects and also interact chemically such that when used in combination will facilitate the cellular uptake of curcumin compared to the amount able to enter into cancer cells when curcumin is used alone. These discoveries address the major limitations seen human clinical trials, in which low or no efficacy is seen at the site of disease in patients.

It has been unexpectedly discovered that curcumin can be stabilized by combining it with one or more antioxidants, or derivatives of antioxidants. As used herein, stabilizing curcumin includes for example increasing the cellular half-life, preventing oxidation, preventing degradation, or preventing deactivation. Examples of suitable antioxidants include resveratrol, catechin, vitamin A, vitamin C, vitamin E, carotenes, flavonoids, chicoric acid, chlorogenic acid cinnamic acid, ellagic acid, ellagitannins gallic acid, gallotannins, propyl gallate, sodium sulfite, sodium metabisulfite, sodium bisulfite, thioglycerol, thioglycollic acid, rosmarinic acid, Salicylic acid, xanthones, eugenol, antioxidant peptides, essential oils and antioxidant fatty acids. Examples of suitable flavonoids include anthoxanthins, flavanones, flavanonols, flavans, isoflavonoids.

Without wishing to be bound by theory, it is believed that the antioxidant properties of catechin and resveratrol increase aqueous stability, cellular uptake, and increased half-life in the body.

In one embodiment, the compositions or formulations of the present invention further includes one or more of the following: a lipophilic carrier, a hydrophilic carrier, a mucoadhesive agent, a solubilizing agent, stabilizer or emulsifier buffer, lubricant, and filler.

In another embodiment, the compositions or formulations of the present invention is formulated as a suppository, a cream, gel, foam, spray, film, sponge, tablet, capsule, emulsion, solution, lotion, suspension, particles, organic polymer, nanoparticles, or a bioadhesive.

In yet another embodiment, the compositions or formulations of the present invention is attached to, incorporated into, or covering a vaginal device or a device that is inserted into oral or anal cavity, or placed in contact with the skin.

In yet another embodiment, the vaginal device, as mentioned above is a tampon, tampon-like device, ring, pessary, tablet, capsule or pellet, sponge, foam, or female condom.

In yet another embodiment, the compositions or formulations of the present invention is in the form of a pellet, tablet, capsule, foam, film, oral rinse, lozenge, or patch. Furthermore, these forms can be placed into the oral cavity or anal cavity. For example, the composition or formulation may be in the form of a suppository for placement into the anal cavity; or a lollipop for placement in an oral cavity.

In yet another embodiment, compositions or formulations of the present invention is attached to, incorporated into, coated onto, or imbedded into a condom.

The compositions, formulations, and composition for use in methods disclosed herein may be used to treat any viral infections. Non-limiting examples of viruses include CMV, HSV, EBV, HIV, HBV, HCV, HTLV, HPV, HHV-8, EBV, or Merkel cell polyomavirus. In another preferred embodiment, the compositions, formulations, and methods disclosed herein may be used to treat oncogenic viruses. An oncogenic virus is a virus that can cause cancer. The compositions or formulations, as described above, can be active against one or multiple pathways implicated in viral infections.

The compositions, formulations, and methods disclosed herein may be used to treat any disease in which uncontrolled cellular proliferation occurs, such as cancers. The compositions or formulations, as described above, can be active against one or multiple pathways implicated in cancer.

The type of cancer applicable to the instant invention is not particularly limited. The compositions, formulations, and methods disclosed herein can be applied to solid cancer, blood cell cancer, and the like. Among these cancers, the solid cancer is a preferable subject to which the invention is applicable.

A non-limiting list of cancers as applicable to the instant invention include glioblastoma, medulloblastoma, leukemia, Hodgkins lymphoma, non-Hodgkins lymphoma, carcinoma, sarcoma, myeloma, prostate cancer, bladder cancer, breast cancer, kidney cancer, pancreatic cancer, anal cancer, oseophegal cancer, colon cancer, skin cancer, bilary cancer, stomach cancer, head and neck cancer, solid carcinoma, squamous cell carcinoma, adenocarcinoma, glioma, high grade glioma, blastoma, neuroblastoma, plasmacytoma, histiocytoma, melanoma, adenoma, hypoxic tumor, myeloma, AIDS-related lymphoma or AIDS-related sarcoma, or metastatic cancer, B cell lymphoma, T cell lymphoma, mycosis fungoides, Hodgkin's Disease, myeloid leukemia, brain cancer, nervous system cancer, squamous cell carcinoma of head and neck, lung cancer, small cell lung cancer, non-small cell lung cancer, ovarian cancer, pancreatic cancer, prostate cancer, skin cancer, liver cancer, melanoma, squamous cell carcinoma of the mouth, squamous cell carcinoma of the throat, squamous cell carcinoma of the larynx, squamous cell carcinoma of the lung, colon cancer, cervical cancer, cervical carcinoma, uterine sarcoma, urethral cancer, endometrial uterine cancer, vaginal cancer, vulvar cancer, epithelial cancer, renal cancer, genitourinary cancer, pulmonary cancer, esophageal carcinoma, large bowel cancer, hematopoietic cancer, testicular cancer, rectal cancer, prostatic cancer, or pancreatic cancer.

The compositions, formulations, and methods disclosed herein may also be used for the treatment of precancer conditions such as cervical and anal dysplasias, other dysplasias, severe dysplasias, hyperplasias, atypical hyperplasias, and neoplasias. The compositions or formulations may also be administered prophylactically to subjects who are at risk for a cancer as described herein.

Methods may be carried out by the procedures described herein, or modification thereof which will be apparent to one skilled in the art.

In another embodiment, the compositions or formulation as described above may be administered alone or in conjunction with radiation or chemotherapeutic agents or surgical excision of the involved cells or lesions.

Preferable examples of the chemotherapeutic agent in the present invention include a cancerocidal agent, an anticancer agent or an antitumor agent (hereinafter referred to collectively as anticancer agent), and specifically the anticancer agent includes bleomycin and derivatives thereof, anthraquinone-based cancerocidal agents including adriamycin and daunomycin, mitomycin and derivatives thereof, actinomycin and derivatives thereof, taxane derivatives such as taxol, camptothecin and derivatives thereof such as irinotecan, cisplatin and derivatives thereof, staurosporine and derivatives thereof, vincristine, streptozotocin, 5-fluorouracil (5-FU) and derivatives thereof, viralbicin and dolastatin, as well as pharmacologically acceptable salts thereof.

The compositions or formulations of the instant invention may be administered in a number of ways depending on whether local or systemic treatment is desired, and on the area to be treated. The therapeutic agent can be provided in any suitable form, generally depending upon the desired administration route. For example, the therapeutic agent can be provided as a salt, a solid, a lyophilized solid, a liquid, a suspension, an aggregate, or a gel. The active compound may be administered in a convenient manner such as by the oral, intravenous (where water soluble), ophthalmic, vaginal, by inhalation, intraperitoneal, intracavity, transdermally intramuscular, intravenous, intranasal, intradermal, subcutaneous, or suppository routes. Depending on the route of administration, the active ingredients of the compositions of the invention as described above containing pharmaceutical compositions may be required to be coated in a material to protect said ingredients from the action of enzymes, acids or other natural conditions.

The composition or formulations of the present disclosure may be encapsulated by lipids. Encapsulation of substrates by liposomes is commonly known in the art. For example, the composition or formulation of the invention may generally be prepared by the process of combining a lipid composition with a solution or suspension of composition or formulations of the present invention under conditions suitable for liposome formation with the desired amount of composition or formulation thereof encapsulated therein.

In addition, the composition could be delivered to a tumor via intralesional delivery using endoscopy, bronchoscopy or colonoscopy or any interventional procedure that can guide a needle or catheter into a body cavity or organ with a tumor mass.

The composition can be used in a method of treatment which includes administering an effective amount of the compositions or formulations to the subject in need thereof. The term "subject" is a concept that encompasses whole individuals, organs, tissues, or cells of humans and non-human animals under the possibility of viral infection, viral progression, viral growth, oncogenic condition, or pre-cancer condition, and includes, for example, tested individuals, and experimental animals. An effective amount is defined as an amount sufficient to achieve a beneficial outcome. In particular, an effective amount may be an amount sufficient to reduce, prevent, suppress, or inhibit the growth or proliferation of, or virus or cancer cells in a subject.

In a preferred embodiment, the effective amount is from about 1 µg to about 2000 µg per kg of body weight per day. The active ingredients (curcumin, resveratrol, and catechin) being present in a ratio of 64:16:200 to 4:1:12.5. The dosage regimen may be adjusted to provide the optimum therapeutic response. For example, several divided doses may be administered daily or the dose may be proportionally reduced as indicated by the exigencies of the therapeutic situation.

The compositions and formulations according to the instant invention may include carriers, thickeners, diluents, buffers, preservatives, surface active agents and the like in addition to the delivery vehicle and/or the therapeutic agent. Pharmaceutical compositions may also include one or more active ingredients such as antimicrobial agents, anti-inflammatory agents, anesthetics, and the like.

In certain aspects, compositions and formulations of the present teachings can be used in a method for delivery to the affected area of the skin in a pharmaceutically acceptable topical carrier. As used herein, a pharmaceutically acceptable topical carrier can be any pharmaceutically acceptable formulation that can be applied to the skin surface for topical, dermal, intradermal, or transdermal delivery of a pharmaceutical or medicament. The combination of a pharmaceutically acceptable topical carrier and a compound of the invention is termed a topical formulation of the invention. Topical formulations of the invention can be prepared by mixing a compound of the invention with a topical carrier according to well-known methods in the art, for example, methods provided by standard reference texts such as, REMINGTON: THE SCIENCE AND PRACTICE OF PHARMACY 1577-1591, 1672-1673, 866-885(Alfonso R. Gennaro ed. 19th ed. 1995); Ghosh, T. K.; et al. TRANSDERMAL AND TOPICAL DRUG DELIVERY SYSTEMS (1997).

The compositions and formulations according to the instant invention suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must preserved against the contaminating action of microorganisms such as bacteria and fungi. The carrier may be a solvent or dispersion medium containing, for example, water, ethanol, polyol (for example, glycerol, propylene glycol, and polyethylene glycol, and the like), suitable mixtures thereof, and vegetable oils. The proper fluidity may be maintained, for example, by the use of a coating such as lecithin, by the maintenance of the required particle size in the case of dispersion and by the use of surfactants. The preventions of the action of microorganisms may be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions may be brought about by the use in the compositions of agents delaying absorption, for example, aluminum monostearate and gelatin.

Sterile injectable solutions are prepared by incorporating the active compositions and formulations in the required amount in the appropriate solvent with various other ingredients enumerated above, as required, followed by filter sterilization. Generally, dispersions are prepared by incorporating the various sterilized active ingredients into sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

It is especially advantageous to formulate parenteral compositions in dosage unit form for ease of administration and uniformity of dosage. Unit dosage form as used herein refers to physically discrete units suited as unitary dosages for a person in need thereof to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health impaired as herein disclosed in detail.

The compositions and formulations of the invention as described above may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparation should contain at least 1% of active compound. The percentage of the compositions and preparations may, of course, be varied and may conveniently be between about 5 to about 80% of the weight of the unit. The amount of active compound in such therapeutically useful compositions is such that a suitable dosage is obtained. A suitable dosage is any dosage sufficient to achieve a beneficial outcome. Preferred compositions or preparations according to the present invention are prepared so that an oral unit dosage form contains between about 10 µg and 1000 µg of active compound.

The tablets, troches, pills, capsules and the like may also contain the following: A binder such as gum agragacanth, acacia, corn starch or gelatin; excipients such as dicalcium phosphate; a disintegrating agent such as corn starch, potato starch, alginic acid and the like; a lubricant such as magnesium stearate; a sweetening agent, such as sucrose, lactose or saccharin or a flavoring agent such as peppermint, oil of wintergreen, or cherry flavoring. When the dosage form is a capsule, it may contain, in addition to materials of the above type, a liquid carrier. Various other materials may be present as coatings or to otherwise modify the physical form of the unit dosage. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

In another aspect, the disclosure relates to a method for increasing pro-apoptotic gene expression in a cell by administering the above-identified composition to a cell. Provided is an *ex vivo* method of increasing pro-apoptotic gene expression in a cell, said method comprising administration of a composition comprising curcumin, catechin and resveratrol in a ratio of 32:8:100 to the cell, wherein said catechin is (-)-epicatechin or a gallic acid conjugate thereof, and wherein said pro-apoptotic gene is selected from the group consisting of CD30, proteasome subunit alpha type 8, and obscurin.

In another aspect, the disclosure relates to a method of decreasing anti-apoptotic gene expression in a cell by administering the above-identified composition to a cell. Provided is an *ex vivo* method of decreasing anti-apoptotic gene expression in a cell, said method comprising administration of a composition comprising curcumin, catechin and resveratrol in a ratio of 32:8:100 to the cell, wherein said catechin is (-)-epicatechin or a gallic acid conjugate thereof, and wherein said anti-apoptotic gene is PEG 10, or rho- associated coiled-coil containing protein kinase 1 (ROCK1).

In another aspect, the disclosure provides an *ex vivo* method of increasing interleukin 2 gene expression in a cell, said method comprising administration of a composition comprising curcumin, catechin and resveratrol in a ratio of 32:8:100 to a cell, and wherein said catechin is (-)-epicatechin or a gallic acid conjugate thereof.

As used herein, the relative amounts of the active ingredients are expressed as a ratio, A:B:C, wherein the values of each component are expressed in units of concentration (µM). Therefore, the active ingredients must be present in a particular amount in relation to the other components.

Reference throughout this specification to "one embodiment", "an embodiment", "one example" or "an example" means that a particular feature, structure or characteristic described in connection with the embodiment or example is included in at least one embodiment of the present embodiments. Thus, appearances of the phrases "in one embodiment", "in an embodiment", "one example", or "an example" in various places throughout this specification are not necessarily all referring to the same embodiment or example. Furthermore, the particular features, structures or characteristics may be combined in any suitable combinations and/or subcombinations in one or more embodiments or examples. In addition, it is appreciated that the figures provided herewith are for explanation purposes to persons ordinarily skilled in the art and that the drawings are not necessarily drawn to scale.

As used herein, the terms "comprises", "comprising", "includes", "including", "has" , "having" , or any other variation thereof, are intended to cover a non-exclusive inclusion. For example, a process, article, or apparatus that comprises a list of elements is not necessarily limited to only those elements but may include other elements not expressly listed or inherent to such process, article, or apparatus.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In this specification, groups of various parameters containing multiple members are described. Within a group of parameters, each member may be combined with any one or more of the other members to make additional sub-groups. For example, if the members of a group are a, b, c, d, and e, additional sub-groups specifically contemplated include any one, two, three, or four of the members, e.g., a and c; a, d, and e; b, c, d, and e; etc.

Throughout this specification, quantities are defined by ranges, and by lower and upper boundaries of ranges. Each lower boundary can be combined with each upper boundary to define a range. The lower and upper boundaries should each be taken as a separate element. Furthermore, one boundary of ranges can be taken independently or in the absence of the other.

Additionally, any examples or illustrations given herein are not to be regarded in any way as restrictions on, limits to, or express definitions of any term or terms with which they are utilized. Instead, these examples or illustrations are to be regarded as being described with respect to one particular embodiment and as being illustrative only. Those of ordinary skill in the art will appreciate that any term or terms with which these examples or illustrations are utilized will encompass other embodiments, which may or may not be given therewith or elsewhere in the specification and all such embodiments are intended to be included within the scope of that term or terms. Language designating such nonlimiting examples and illustrations includes, but is not limited to: "for example", "for instance", "e.g.", and "in one embodiment".

The present disclosure may be better understood with reference to the examples, set forth below. The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### EXAMPLES

The present invention is illustrated in further details by the following non-limiting examples.

### Example 1

### Screening

First databases for plant polyphenols were screened and searched, based on reported effects, and chemical structures to associate with curcumin. Candidate compounds were examined by using a cell viability assay (WST-1 assay) in the well-known HPV-18 positive cancer cell line HeLa. A dose response curve and IC50 were obtained using the Graphpad Prism statistical analysis program (Fig 1). Then the compounds that showed anticancer properties were selected for the assessment of Combination Index (CI) using a method and software (CompuSyn) developed by Chou and Talalay. This method used cell viability to determine if individual drugs in a combination function in synergistic, additive or antagonistic manner to one another. At the beginning we tested the combination index for two drugs at a time then subsequently three drugs were combined. To determine the CI we treated HeLa cells with different doses of each of the drugs alone as well as in combinations of 4 or 5 concentrations of each. After 96 hours of treatment, cell viability assay was performed. The data obtained from this WST-1 assay were converted to fractions affected (Fa) and analyzed by the CompuSyn program. The present disclosure shows that three drugs, Curcumin, Epicatechin, and Resveratrol, in stoichiometric combinations, Curcumin:Epicatechin:Resveratrol: 32:8:100, 16:4:50, 8:2:25, and 4:1:12.5 were synergistic in and optimal in killing HeLa cells in culture. See Figures 2 and 3.

### Example 2

### A combination of Curcumin, Epicatechin, and Resveratrol causes rapid suppression of Phospho-NFkB (P-NFkB), NFkB, P-Rb and restoration of p53 in HeLa cells in 8 h.

Discrete signaling proteins mediate transformation, survival, and proliferation of various types of cancer cells. The composition according to the invention disrupts signaling or causes overexpression of key signaling proteins which are not specific to a cancer cell type. Rapid changes occur even before any cell degeneration is visible (Figure 4). Thus, NF-kB is rapidly inhibited within 8 hours of treatment. Most of the other combinations yielded no significant difference in band intensity with respect to the vehicle-treated control. It should be noted that 50 µM curcumin also causes similar effects on P53, P-NF-kB, and HPVE6, but a lower concentration of curcumin (32 µM) does not produce such rapid suppression of gene products unless combined with E and R in the specific ratio used in Curc32+. The quantification of bands was performed using the ImageJ program and plotted for p53 and NFkB by normalizing against beta actin intensity. P-Rb was normalized to total Rb and P-NFkB to total NFkB.

Most notably the cell cycle inhibitor p53 is dramatically increased compositions and formulations as disclosed herein. Meanwhile, the botanical compounds inhibit the phosphorylated Rb fraction (pRb), which prevents cell cycle progression. This is demonstrated by a progressive decrease in the pRb/RB with the double then triple combinations (figure 4). Additionally, the cell-transforming HPV protein E6 is dramatically inhibited by Curc 32+. Taken all together, Figures 1 and 4 demonstrate that each individual compound (curcumin, epicatechin, and resveratrol) reverses the malignant phenotype of cervical cancer cells. However, the triple combination has synergistic effect modulating the key pathways at a lower concentration of curcumin. This correlates with the cytoxicity data. Furthermore, Curcumin causes suppression of the cell-transforming HPV antigen E6: Immunocytochemical analysis revealed detectable expression of HPV-E6 in four cervical cancer cell lines, HeLa, ME-180, SiHa, and SW756, but not in the human fibroblast cells (Figure 5). Curcumin treatment caused a rapid and significant decrease in the expression of HPV-E6 in all the four cell lines, prior to cell death. RT-PCR data confirms that curcumin down regulates mRNA (Figure 4). Taken together, the data show that the compositions and formulations as disclosed herein are potent and alter key pathways cancers cells use to survive and grow, the disclosed triple combination synergistically reverse the malignant phenotype in cancer cells prior to cell death. In addition, the present disclosure show that the composition or formulation disclosed herein also has synergic effects in down regulating the essential HPV E6 protein, used by this virus to replicate.

### Example 3

### Prototype Cream

To enhance the antiviral and anticancer properties of the composition or formulation disclosed herein and to further address the low systemic bioavailability issue seen in clinical trials, the present disclosure provides a method for local delivery as HPV infection and the associated cancers occur on epithelial surfaces, like the vaginal canal and cervix. Vaginal curcumin formulation: Intravaginal formulation containing 2%, 5%, 10%, and 20% (w/w) curcumin were prepared by mixing curcumin powder with a commercially available topical oil-in-water cream base called Vanicream (Pharmaceutical Specialties, Inc., Rochester, MN). These formulations were named as Vacurin-2, Vacurin-5, Vacurin-10, and Vacurin-20, respectively. Applicants' tests showed that 5 g of Vacurin occupied a volume of 5 ml. Therefore, we expressed the concentrations of curcumin in Vacurin-mixture as 20% (w/v). The two components were thoroughly mixed using a spatula and the homogeneity of the mixture was verified by visualizing curcumin fluorescence at 530 nm using a Leica Laser Confocal Scanning System (Exton, PA) TCS SP2. Similarly, epicatechin, and resveratrol where tested for miscibility in the Vacurin mixture, compounding formulations with molarities in the optimal dosage ratio established in Applicants' combination experiments.

### Example 4

### Prototype Lollipop

Similarly, for local oral delivery of the composition or formulation as disclosed herein a formulation for a medicinal lollipop of curcumin, epicatechin, and resveratrol were developed on optimized proportion of 64:16:200 or 32: 8:100. The compounds are placed in a sorbitol base, mixed, and heated to form a solvent. Once uniformity in the solvent is achieved, the mixture is then allowed to cool in a mold.

### Example 5

### Curcumin, Epicatechin, and Resveratrol eliminate HeLa cells in a dose dependent manner.

Cells were treated with indicated concentrations of each drug for 96 hours and then subjected to WST-1 assays. Carrier-treated HeLa cells were used as control. Treatment of primary human fibroblast yields some cell death only at high concentrations of curcumin. The data were analyzed using the Graphpad Prism software. The IC50 value for each drug is indicated in the graph. See figure 1.

### Example 6

### Synergistic anti-cancer activities of drug combinations in HeLa cells

Curcumin, Epicatechin, and Resveratrol alone or in combinations were used to treat HeLa cells at indicated doses. After 96 hours of treatment, WST-1 assay was carried out to estimate cell viability. Carrier-treated cells were used as control. The fractions of cells affected by these treatments were plotted using Graphpad Prism. Results demonstrate that the combinations of the three anti-cancer agents have better killing activities than the individual drugs or the combinations of two drugs. See figure 2.

### Example: 7

The composition of curcumin, epicatechin, and resveratrol in increasing or decreasing doses of the proportion 32:8:100 was named Curc 32+. Dilutions of this stock were made based on curcumin concentration and tested for its ability to eliminate the human HPV (+) cervical cancer cell line, Hela, and the murine epithelial HPV (+) carcinoma cell line, TC-1.

### Curc 32+ is more potent in eliminating both HeLa and TC-1 cells.

It was determined that 4 µM curcumin in Cur 32+ was required to kill 50% of HeLa cells, It was determined that 4 µM of curcumin in Curc 32+was required to kill 50% of HeLa cells, while the IC50 of curcumin alone is 17 µSimilarly, 13 µM of curcumin in Curc 32+ was able to kill 50% of TC-1 cells, while 354 µM of curcumin was needed when this compound was used alone.

IC50 of curcumin alone and Curc 32+.

| Cell Lines | Curcumin Alone IC50(µM) | Curcumin in Curc 32+^{∗∗} IC50(µM) |
|---|---|---|
| HeLa | 17 | 4 |
| TC-1 | 35 | 13 |

| | | |
|---|---|---|
| ^{∗}HPV(+) mouse carcinoma cells that have been used in the shown mouse model ^{∗∗}The IC50 of curcumin decreases by several fold after adding epicatechin and resveratrol. | | |

### Example 8

### A combination of Curcumin, Epicatechin, and Resveratrol causes rapid suppression of Phospho-NFkB (P-NFkB), NFkB, P-Rb, and restoration of p53 in HeLa cells in 8 hours

A combination of Curcumin, Epicatechin, and Resveratrol causes rapid suppression of Phospho-NFkB (P-NFkB), NFkB, P-Rb, and restoration of p53 in HeLa cells in 8 h. The quantification of bands was performed using the ImageJ program and plotted for p53 and NFkB by normalizing against betaactin intensity. P-Rb was normalized to total Rb and P-NFkB to total NFkB. See figure 3.

### Example 9

### Curcumin treatment for 8 hours dramatically inhibits E6 expression, but only at a higher concentration (50 µM) than in Curc 32+. The amount of curcumin that inhibited E6 in Curc 32+is 32 µM.

HeLa cells were cultured in 6 well plates and treated with either carrier or curcumin (50 µM) for 8 h. RNA was isolated using the Trizol reagent and treated with DNase I to remove contaminating DNA. Using primers specific for HPV18 E6 or GAPDH, semi-quantitative reverse transcriptase PCR (RT-PCR) was performed on cDNA samples obtained from both treatments. Equal volumes of the PCR reactions were separated on a 1.5 % agarose-ethidium bromide gel. (A) Two amplified products were obtained- one of ~450 bps corresponding to full-length E6 and a second of ~ 280 bps representing the spliced E6^{∗} product. A low exposure image of the spliced PCR products is also shown below. (B) The intensity if each band was quantified, normalized to GAPDH, and plotted in graphs. See figure 4.

### Example 10

### Immunocvtochemical analysis revealed detectable expression of the HPV oncogenic protein E6

Immunocytochemical analysis revealed detectable expression of the HPV oncogenic protein E6 in all four cervical cancer cell lines, HeLa, ME-180, SiHa, and SW756, but not in the human fibroblast cells (only graph is shown). Curcumin (50 µM) treatment caused a significant decrease in the expression of HPV-E6 in all the four cell lines (only HeLa cells images are shown), demonstrating curcumin has antiviral properties reversing the overexpression of the oncogenic protein E6. See figure 5.

### Example 11

### The E6 protein is expressed by multiple HPV(+) cell lines.

Immunocytochemical analysis revealed detectable expression of the HPV oncogenic protein E6 in all four cervical cancer cell lines, HeLa, ME-180, SiHa, and SW756, but not in the human fibroblast cells. See figure 6.

### Example 12

### Curc 32+ treatment inhibits E6 expression in mouse HPV(+) TC-1 cells

The TC-1 tumor cells were originally created by immortalizing primary lung epithelial cells from C57BL/6 mice with the amphotrophic retrovirus vector LXSN16E6E7 and subsequently transformed with the pVEJB plasmid expressing human c-Ha-ras. These cells express both E6 and E7. See figure 7a and 7b.

### Example 13

### Intra-tumor administration of Curc 32+ kills tumor cells.

A chimeric cancer mouse model was used to evaluate the in vivo effects of Curc 32+. **(A)** C57BL/6 mice had 50,000 TC-1 cancer cells implanted in the neck of each mouse. Neck tumors cause death at 4-5 weeks. For the intralesional experiments, TC-1 tumors were allowed to grow for about 2 weeks then mice received two doses of (64:16:200) 72 hours apart. See Figure 8a. The mice were imaged 48 hours after the second injection to observe a dramatic reduction of tumor size (Figure 8a). In another experiment, the mice were sacrificed 5 days after the second dose. **See** **Figure 8b****.** H&E stained section of TC-1 tumor in a mice with no treatment, HPV(+) carcinoma cells form a solid mass with no areas of necrosis. Figure 8b **(C)** Mice treated with two doses of intralesional Curc 64+ show extensive area of tumor necrosis near the injection track, 5 days post treatment (tissue loss and extended necrosis). Figure 8b **(D)** High magnification image of (C) confirms the presence of tumor cell necrosis.

### Example 14

### Systemic (i.p.) Curc 32+ administration prolongs the life of mice implanted with TC-1 tumors in the neck.

Clinical trials with Curcumin, shows that curcumin achieves low tumor concentrations systemically and poor efficacy. To test if the composition of the current invention can achieve systemic activity, we examine the systemic effects of Curc 32+ on the TC-1 mouse tumor model. On day 1, TC-1 cells (50,000) were implanted subcutaneously in the neck of two groups of C57BL6 mice (three per group). The mice were then treated with intra-peritoneal infusion of vehicle or Curc 32+ every 72 hours, starting 72 hours after cell implantation. Compared to vehicle-treated mice (light grey or red line), the Curc 32+-treated mice showed prolonged survival (dark grey or blue line) (p = 0.02). See Figure 9. These data is in contrast to clinical trial results in which no effect on mortality was seen in human studies.

### Example 15

### Genome-wide microarray analysis

Genome-wide microarray analysis was performed to identify expression levels of several cancer regulatory and immune modulatory genes. See Figures 10-15. Analysis was done on 30,000 genes in HeLa cells after exposure to vehicle, curcumin alone, epicatechin alone and resveratrol. Groups were compared to Curc 32+ treated cells. Exclusively, Curc 32+ treatment of HeLa cells for 8 hours caused a dramatic increase in the expression of *CD30, Prosome alpha* 8, and *Obscurin* genes, which yield proapoptotic proteins (Figure 10, 11, 12). Simultaneously, a dramatic suppression of the genes *ROCK1* and *PEG10* that yield anti-apoptotic products was observed exclusively in the Curc 32+-treated samples (Figure 13, 14). Finally, a dramatic increase in the expression of the *IL2* gene that yields the anti-tumor cytokine IL-2 was observed exclusively in the Curc 32+-treated samples (Figure 15). The microarray data shows that the triple combination of compounds, in portions disclosed herein, derive function biological as a new entity, as essential genes involved in cancer control and immunity can be affected to reverse the cancer phenotype. The genes identified in the microarray analysis were not affected by the individual compounds when used alone. The present disclosure provides further evidence that the compositions and formulations disclosed herein target several key genes to produce a novel composition that activates unique pathways not done by individual compounds. This shows some mechanistic data to support potent and synergistic antiviral and antitumor effects seen in the disclosed *in vitro* and *in vivo* experiments.

### Example 16

### The Curc 32+formulation significantly enhances cellular uptake of curcumin and stabilizes curcumin in an aqueous environment.

HeLa cells where incubated for 4 hours with either curcumin at 32 µM or Curc 32+ (consisting of curcumin 32 µM + epicatechin 8 µM+ resveratrol 100 µM). Curcumin fluorescence s at an excitation wavelength of 450 nm, therefore, fluorescence microscopy was used to track curcumin uptake into HeLa cells (Figure 16). When using the Curc 32+ formulation, curcumin's uptake was significantly increased by 17.8% compared to the uptake of curcumin when used alone. (p < 0.0001; t-test). This is a new property not previously attributed to epicatechin and/or resveratrol.

Next, using identical conditions and amounts as the 4 hours experiment, curcumin alone and Curc 32+ were left in solution (culture medium) overnight at 37 °C and then added to cells. The cellular uptake of curcumin was increased approximately by 25% when curcumin was left in the form Curc 32+ as compared to when curcumin was left alone (p < 0.0001). As equal amounts of curcumin were used in the 4-hour and 24-hour experiments, these results indicate that Curc 32+ may also protect curcumin from oxidation while in an aqueous type environment. Curcumin is a very potent antioxidant; it is quickly oxidized *via* interactions with many redox components of a living cell and the environment. Even when it is left in an aqueous solution it reacts with oxygen. Without wishing to be bound by theory, it is thought that when curcumin participates in these redox reactions it loses its anticancer properties. This is one of the reasons curcumin is not stable in blood, it gets degraded and is not able to reach diseased tissue. To overcome the limitations of getting curcumin to the target tissue in a clinical setting, many other approaches have been attempted such as altering the natural structure of curcumin, however, this can decrease the biologic function of curcumin or such curcumin derivatives could become less specific and toxic affecting normal cells. Other approaches include making more complex entities like nanoparticles. The approach disclosed herein is much simpler to manufacture compared to these other methods and the presently disclosed method unexpectedly enhances curcumin's beneficial properties.

### Example 17

### Preparation of Curc 64+ Liposome using 20% Sov Phosphatidylcholine

First, a solution of C-E-R (1280: 320: 4000) (µM) was prepared. Appropriate weights of curcumin (C), epicatechin-gallate (E), and resveratrol (R) were weighed out and dissolved to correspond to a final concentration of 1.28 mM, 320 µM and 4 mM respectively. Curcumin and resveratrol were initially dissolved in DMSO and epicatechin was dissolved in deionized water. Appropriate volumes of each were added to a fixed volume of filtered phosphate buffered saline corresponding to the above-mentioned concentrations. An appropriate mass of soy phospholipid mixture from Avanti Polar Lipids (catalog# 541601G) was added to the solution of C-E-R to maintain a mass ratio of 4:1 of lipid mixture: C-E-R. Vigorous sonication was performed on the mixture at 4 °C to disperse the mixture and form an emulsion. Solid precipitate was removed by centrifugation. The mixture was stored at 4 °C after purging thoroughly with nitrogen. 200 µL of the solution was intra-peritoneally injected into each mouse to give an approximate final concentration of 64+ µM C-E-R in the total mouse body fluid of 4 ml. 64+ µM means that curcumin:catechin:resveratrol is present in the following concentration/ratio (µM) 64:16:200. At the specified times, blood was collected from the heart (through terminal surgery) in heparinized tubes (total volume ~600 µl). The tubes were centrifuged to pellet down cells and collect the supernatant (plasma) in a labeled, chilled tube. Exactly, 300 µl of this plasma from each mouse was extracted with acetonitrile: water (1:1) (1.2 ml) by vortexing vigorously. The mixture was centrifuged, the supernatant transferred to a fresh Falcon tube and evaporated at 45 °C under a stream of nitrogen. The residue left was extracted twice with 0.5 ml acetonitrile and the pooled acetonitrile extract (total 1 ml) was evaporated under a stream of nitrogen. The residue left was dissolved in acetonitrile (40µl) and a volume of 25 µl was injected into the HPLC analyzer fitted with a C18 reverse-phase column. The column was eluted using the following solvents: acetonitrile and ammonium acetate (pH 4.5). Curcumin was eluted as a triplet at a retention time of -20 min. See Figure 17.

In summary, the present disclosure provides a unique method to enhance the activity of curcumin. The disclosed composition is biologically more active demonstrating strong synergy, functions to activate multiple novel genes. The disclosure further shows that the inventive composition increases cellular uptake of curcumin and able to stabilize curcumin in an aqueous environment. All these enhanced properties are accomplished by a method that is much simpler to manufacture and lower in cost. The composition and formulations of the present disclosure have novel properties not previously reported in any prior art or publication.

## Claims

1. A pharmaceutical composition comprising curcumin, catechin and resveratrol, wherein said curcumin, catechin and resveratrol are present in a ratio of 32:8:100, and wherein said catechin is (-)-epicatechin or a gallic acid conjugate thereof.

2. Composition according to claim 1, further comprising a lipophilic carrier, a hydrophilic carrier, a muco-adhesive agent, a solubilizing agent, stabilizer or emulsifier buffer, lubricant, filler, alone or in combination.

3. Composition according to claim 1 or 2, wherein said composition is formulated as a suppository, a cream, gel, foam, spray, film, sponge, tablet, capsule, emulsion, solution, lotion, nanofiber, suspension, particles, nanoparticles, a bio-adhesive, or an oral rinse.

4. Composition according to any one of claims 1-3, wherein said composition is attached to, incorporated into, or covering a vaginal device or a device that is inserted into oral or anal cavity, or placed in contact with the skin, preferably wherein said vaginal device is a tampon, tampon-like device, ring, pessary, tablet, capsule or pellet, sponge, foam, or female condom.

5. Composition according to any one of claims 1-3, wherein said composition is attached to, incorporated into, coated onto, or embedded into a condom.

6. A pharmaceutical composition according to any one of claims 1-5, for use in a method of treating a viral infection caused by human papilloma virus (HPV), said method comprising administering to a cell infected with a virus said composition to inhibit viral replication.

7. A pharmaceutical composition according to any one of claims 1-5, for use in a method of treating cancer caused by human papilloma virus (HPV) by suppressing cancer cell proliferation, said method comprising administering to a cancerous cell said composition to inhibit cancer cell proliferation.

8. Composition for use according to claim 7, wherein said cancer caused by HPV is cervical cancer, oropharyngeal cancer, anal cancer, penile cancer, vaginal cancer or precancerous lesions of the cervix.

9. An *ex vivo* method of increasing pro-apoptotic gene expression in a cell, said method comprising administration of a composition comprising curcumin, catechin and resveratrol in a ratio of 32:8:100 to the cell, wherein said catechin is (-)-epicatechin or a gallic acid conjugate thereof, and wherein said pro-apoptotic gene is selected from the group consisting of CD30, proteasome subunit alpha type 8, and obscurin.

10. An *ex vivo* method of decreasing anti-apoptotic gene expression in a cell, said method comprising administration of a composition comprising curcumin, catechin and resveratrol in a ratio of 32:8:100 to the cell, wherein said catechin is (-)-epicatechin or a gallic acid conjugate thereof, and wherein said anti-apoptotic gene is PEG 10, or rho-associated coiled-coil containing protein kinase 1 (ROCK1).

11. An *ex vivo* method of increasing interleukin 2 gene expression in a cell, said method comprising administration of a composition comprising curcumin, catechin and resveratrol in a ratio of 32:8:100 to a cell, and wherein said catechin is (-)-epicatechin or a gallic acid conjugate thereof.

12. An *ex vivo* method of increasing cellular uptake of curcumin, said method comprising providing a mixture of curcumin, catechin and resveratrol in a ratio of 32:8:100, wherein said catechin is (-)-epicatechin or a gallic acid conjugate thereof, and applying said mixture to a cell.

## Patentansprüche

1. Pharmazeutische Zusammensetzung, umfassend Curcumin, Catechin und Resveratrol, wobei das Curcumin, Catechin und Resveratrol in einem Verhältnis von 32:8:100 vorhanden sind, und wobei das Catechin (-)-Epicatechin oder ein Gallensäurekonjugat davon ist.

2. Zusammensetzung nach Anspruch 1, ferner umfassend einen lipophilen Träger, einen hydrophilen Träger, ein mukoadhäsives Mittel, ein Lösungsmittel, Stabilisator oder Emulgatorpuffer, Gleitmittel, Füllstoff, allein oder in Kombination.

3. Zusammensetzung nach Anspruch 1 oder 2, wobei die Zusammensetzung als ein Zäpfchen, eine Creme, Gel, Schaum, Spray, Film, Schwamm, Tablette, Kapsel, Emulsion, Lösung, Lotion, Nanofaser, Suspension, Partikel, Nanopartikel, ein Biokleber oder eine Mundspülung formuliert ist.

4. Zusammensetzung nach einem der Ansprüche 1 - 3, wobei die Zusammensetzung an einer Vaginalvorrichtung oder einer Vorrichtung, die in die Mund- oder Analhöhle eingeführt wird, oder in Kontakt mit der Haut platziert ist, angebracht ist, in dieser enthalten ist oder diese bedeckt, vorzugsweise wobei die Vaginalvorrichtung ein Tampon, eine tamponartige Vorrichtung, ein Ring, ein Pessar, eine Tablette, eine Kapsel oder ein Pellet, ein Schwamm, ein Schaum oder weibliches Kondom ist.

5. Zusammensetzung nach einem der Ansprüche 1 - 3, wobei die Zusammensetzung an einem Kondom angebracht, in diesem enthalten, mit beschichtet oder in dieses eingebettet ist.

6. Pharmazeutische Zusammensetzung nach einem der Ansprüche 1 - 5 zur Verwendung in einem Verfahren zum Behandeln einer viralen Infektion, verursacht durch das humane Papillomvirus (HPV), das Verfahren umfassend Verabreichen der Zusammensetzung an eine Zelle, die mit einem Virus infiziert ist, um die Virusreplikation zu hemmen.

7. Pharmazeutische Zusammensetzung nach einem der Ansprüche 1 - 5 zur Verwendung in einem Verfahren zum Behandeln von Krebs, verursacht durch das humane Papillomvirus (HPV), durch Unterdrücken der Krebszellproliferation, das Verfahren umfassend Verabreichen der Zusammensetzung an eine Krebszelle, um die Krebszellproliferation zu hemmen.

8. Zusammensetzung zur Verwendung nach Anspruch 7, wobei der Krebs, verursacht durch HPV, Gebärmutterhalskrebs, oropharyngeale Krebs, Analkrebs, Peniskrebs, Vaginalkrebs oder präkanzeröse Läsionen der Zervix ist.

9. Ex-vivo-Verfahren zum Erhöhen pro-apoptotischer Genexpression in einer Zelle, das Verfahren umfassend Verabreichung einer Zusammensetzung, umfassend Curcumin, Catechin und Resveratrol in einem Verhältnis von 32:8:100 an die Zelle, wobei das Catechin (-)-Epicatechin oder ein Gallensäurekonjugat davon ist, und wobei das pro-apoptotische Gen ausgewählt ist aus der Gruppe, bestehend aus CD30, Proteasom-Untereinheit Alphatyp 8, und Obscurin.

10. Ex-vivo-Verfahren zum Verringern anti-apoptotischer Genexpression in einer Zelle, das Verfahren umfassend Verabreichung einer Zusammensetzung, umfassend Curcumin, Catechin und Resveratrol in einem Verhältnis von 32:8:100 an die Zelle, wobei das Catechin (-)-Epicatechin oder ein Gallensäurekonjugat davon ist, und wobei das anti-aptotische Gen PEG 10, oder Rho-assoziierte Doppelwendel enthaltende Proteinkinase 1 (ROCK1) ist.

11. Ex-vivo-Verfahren zum Erhöhen der Interleukin-2-Genexpression in einer Zelle, das Verfahren umfassend Verabreichung einer Zusammensetzung, umfassend Curcumin, Catechin und Resveratrol in einem Verhältnis von 32:8:100 an eine Zelle, und wobei das Catechin (-)-Epicatechin oder ein Gallsäurekonjugat davon ist.

12. Ex-vivo-Verfahren zum Erhöhen der zellulären Aufnahme von Curcumin, das Verfahren umfassend Bereitstellen einer Mischung aus Curcumin, Catechin und Resveratrol in einem Verhältnis von 32:8:100, wobei das Catechin (-)-Epicatechin oder ein Gallensäurekonjugat davon ist, und Aufbringen der Mischung auf eine Zelle.

## Revendications

1. Composition pharmaceutique comprenant de la curcumine, de la catéchine et du resvératrol, dans laquelle lesdites curcumine, catéchine et resvératrol sont présentes dans un rapport de 32:8:100, et dans laquelle ladite catéchine est la (-)-épicatéchine ou un conjugué d'acide gallique de celle-ci.

2. Composition selon la revendication 1, comprenant en outre un support lipophile, un support hydrophile, un agent muco-adhésif, un agent solubilisant, un tampon stabilisateur ou émulsifiant, un lubrifiant, une charge, seuls ou en combinaison.

3. Composition selon la revendication 1 ou 2, dans laquelle ladite composition est formulée sous la forme d'un suppositoire, d'une crème, d'un gel, d'une mousse, d'un spray, d'un film, d'une éponge, d'un comprimé, d'une capsule, d'une émulsion, d'une solution, d'une lotion, d'une nanofibre, d'une suspension, de particules, de nanoparticules, d'un bioadhésif ou d'un bain de bouche.

4. Composition selon l'une quelconque des revendications 1 à 3, dans laquelle ladite composition est fixée à, incorporée dans, ou recouvre un dispositif vaginal ou un dispositif qui est inséré dans la cavité orale ou anale, ou placé en contact avec la peau, de préférence dans laquelle ledit dispositif vaginal est un tampon, un dispositif de type tampon, un anneau, un pessaire, un comprimé, une gélule ou une pastille, une éponge, une mousse, ou un préservatif féminin.

5. Composition selon l'une quelconque des revendications 1 à 3, dans laquelle ladite composition est attachée à, incorporée dans, revêtue sur, ou incorporée dans un préservatif.

6. Composition pharmaceutique selon l'une quelconque des revendications 1 à 5, destinée à être utilisée dans une méthode de traitement d'une infection virale provoquée par le virus du papillome humain (HPV), ladite méthode comprenant l'administration à une cellule infectée par un virus de ladite composition pour inhiber la réplication virale.

7. Composition pharmaceutique selon l'une quelconque des revendications 1 à 5, destinée à être utilisée dans une méthode de traitement du cancer provoqué par le virus du papillome humain (HPV) par suppression de la prolifération des cellules cancéreuses, ladite méthode comprenant l'administration à une cellule cancéreuse de ladite composition pour inhiber la prolifération des cellules cancéreuses.

8. Composition à utiliser selon la revendication 7, dans laquelle ledit cancer causé par le HPV est un cancer du col de l'utérus, un cancer oropharyngé, un cancer anal, un cancer du pénis, un cancer vaginal ou des lésions précancéreuses du col de l'utérus.

9. Procédé *ex vivo* pour augmenter l'expression d'un gène pro-apoptotique dans une cellule, ledit procédé comprenant l'administration à la cellule d'une composition comprenant de la curcumine, de la catéchine et du resvératrol dans un rapport de 32:8:100, dans lequel ladite catéchine est la (-)-épicatéchine ou un conjugué d'acide gallique de celle-ci, et dans lequel ledit gène pro-apoptotique est choisi dans le groupe constitué par CD30, la sous-unité alpha de type 8 du protéasome et l'obscurine.

10. Procédé *ex vivo* de diminution de l'expression du gène anti-apoptotique dans une cellule, ledit procédé comprenant l'administration à la cellule d'une composition comprenant de la curcumine, de la catéchine et du resvératrol dans un rapport de 32:8:100, dans lequel ladite catéchine est la (-)-épicatéchine ou un conjugué d'acide gallique de celle-ci, et dans lequel ledit gène anti-apoptotique est le PEG 10, ou la protéine kinase 1 contenant une bobine hélicoïdale associée à la rho (ROCK1).

11. Procédé *ex vivo* pour augmenter l'expression du gène de l'interleukine 2 dans une cellule, ledit procédé comprenant l'administration d'une composition comprenant de la curcumine, de la catéchine et du resvératrol dans un rapport de 32:8:100 à une cellule, et dans lequel ladite catéchine est la (-)-épicatéchine ou un conjugué d'acide gallique de celle-ci.

12. Procédé *ex vivo* pour augmenter l'absorption cellulaire de curcumine, ledit procédé comprenant la fourniture d'un mélange de curcumine, de catéchine et de resvératrol dans un rapport de 32:8:100, dans lequel ladite catéchine est la (-)-épicatéchine ou un conjugué d'acide gallique de celle-ci, et l'application dudit mélange à une cellule.
